# EUROPEAN PATENT APPLICATION

(11) **EP 4 848 306 A1**
(43) Date of publication of application: **02.12.2026**
(21) Application number: 26178709.7
(22) Date of filing: 13.05.2026
(51) Int. Cl.: G16H 40/67, A61B 5/00, A61B 5/02, A61B 5/024, G16H 50/20

(54) **TOKENIZED REPRESENTATION OF SENSOR DATA FOR WEARABLE COMPUTING DEVICE**

(30) Priority: 28.05.2025 US 202519220813
(71) Applicant: Google LLC, Mountain View, CA 94043 (US)
(72) Inventor: Tailor, Shyam Anil, London, N1C 4AG (GB)
(74) Representative: Maikowski & Ninnemann Patentanwälte Partnerschaft mbB

(57) **Abstract**

A method for controlling a wearable computing device includes receiving, via a controller, sensor data from one or more sensors of the wearable computing device. The method also includes generating, via a machine-learned tokenization model programmed in the controller on-board the wearable computing device, tokenized sensor data based on the sensor data. The tokenized sensor data includes the sensor data compressed into a compressed bitstream. In addition, the method includes transmitting, via the controller on-board the wearable computing device, the tokenized sensor data to a remote computing device.

## Description

### FIELD

The present disclosure relates generally to a wearable computing device, and more particularly, to tokenizing sensor data for the wearable computing device so as to facilitate offloading the sensor data from the wearable computing device.

### BACKGROUND

Advances in sensors and wearable technologies have made it increasingly possible for individuals to collect data about themselves. Further, artificial intelligence systems increasingly include large foundational machine-learned models which have the capability to provide health assessments and/or recommendations for the user based on the collected sensor data. However, wearable technologies may lack computational resources available to store and/or implement each of the distinct artificial intelligence systems for performing the specific health assessments and/or recommendations.

### SUMMARY

Aspects and advantages of embodiments of the present disclosure will be set forth in part in the following description, or can be learned from the description, or can be learned through practice of the embodiments.

In an aspect, the present disclosure is directed to a method for controlling a wearable computing device. The method includes receiving, via a controller, sensor data from one or more sensors of the wearable computing device. The method also includes generating, via a machine-learned tokenization model programmed in the controller on-board the wearable computing device, tokenized sensor data based on the sensor data. The tokenized sensor data includes the sensor data compressed into a compressed bitstream. In addition, the method includes transmitting, via the controller on-board the wearable computing device, the tokenized sensor data to a remote computing device.

In another aspect, the present disclosure is directed to a wearable computing device. The wearable computing device includes a controller comprising at least one processor. The at least one processor is configured to perform a plurality of operations. The plurality of operations includes receiving sensor data from one or more sensors of the wearable computing device. The plurality of operations also includes generating, via a machine-learned tokenization model programmed in the controller, tokenized sensor data based on the sensor data. The tokenized sensor data includes the sensor data compressed into a compressed bitstream. In addition, the plurality of operations includes transmitting the tokenized sensor data to a remote computing device.

Other example aspects of the present disclosure are directed to other systems, methods, apparatuses, tangible non-transitory computer-readable media, and devices for performing functions described herein. These and other features, aspects, and advantages of various implementations will become better understood with reference to the following description and appended claims. The accompanying drawings, which are incorporated in and constitute a part of this specification, illustrate implementations of the present disclosure and, together with the description, help explain the related principles.

### BRIEF DESCRIPTION OF THE DRAWINGS

A full and enabling disclosure of the present invention, including the best mode thereof, directed to one of ordinary skill in the art, is set forth in the specification, which makes reference to the appended figures, in which:
FIG. 1 illustrates a perspective view of an embodiment of a wearable computing device according to the present disclosure;
FIG. 2 illustrates a schematic diagram of computing elements of the wearable computing device of FIG. 1;
FIG. 3 illustrates a schematic diagram of an embodiment of a computing environment according to the present disclosure;
FIG. 4 illustrates a block diagram of an embodiment of a server system of a wearable computing device according to the present disclosure;
FIGS. 5A-5B illustrate block diagrams of embodiments of a tokenization module of a wearable computing device according to the present disclosure; and
FIG. 6 illustrates aa flow diagram of an embodiment of a method for offloading tokenized sensor data from a wearable computing device according to the present disclosure.

Reference numerals that are repeated across plural figures are intended to identify the same features in various implementations.

### DETAILED DESCRIPTION

Reference now will be made in detail to embodiments, one or more examples of which are illustrated in the drawings. Each example is provided by way of explanation of the embodiments, not limitation of the present disclosure. In fact, it will be apparent to those skilled in the art that various modifications and variations can be made to the embodiments without departing from the scope or spirit of the present disclosure. For instance, features illustrated or described as part of one embodiment can be used with another embodiment to yield a still further embodiment. Thus, it is intended that aspects of the present disclosure cover such modifications and variations.

### Overview

Repeated use of reference characters and/or numerals in the present specification and/or figures is intended to represent the same or analogous features, elements, or operations of the present disclosure. Repeated description of reference characters and/or numerals that are repeated in the present specification is omitted for brevity.

As referred to herein, the terms "includes" and "including" are intended to be inclusive in a manner similar to the term "comprising." As referenced herein, the terms "or" and "and/or" are generally intended to be inclusive, that is, "A or B" or "A and/or B" are each intended to mean "A or B or both." As referred to herein, the terms "first," "second," "third," and so on, can be used interchangeably to distinguish one component or entity from another and are not intended to signify location, functionality, or importance of the individual components or entities. As referenced herein, the terms "couple," "couples," "coupled," and/or "coupling" refer to chemical coupling (e.g., chemical bonding), communicative coupling, electrical and/or electromagnetic coupling (e.g., capacitive coupling, inductive coupling, direct and/or connected coupling, etc.), mechanical coupling, operative coupling, optical coupling, and/or physical coupling.

As referenced herein, the term "system" can refer to hardware (e.g., application specific hardware), computer logic that executes on a general-purpose processor (e.g., a central processing unit (CPU)), and/or some combination thereof. In some embodiments, a "system" described herein can be implemented in hardware, application specific circuits, firmware, and/or software controlling a general-purpose processor. In some embodiments, a "system" described herein can be implemented as program code files stored on a storage device, loaded into a memory, and executed by a processor, and/or can be provided from computer program products, for example, computer-executable instructions that are stored in a tangible computer-readable storage medium (e.g., random-access memory (RAM), hard disk, optical media, magnetic media).

As mentioned, advances in sensors and wearable technologies have made it increasingly possible for individuals to collect data about themselves. However, gaining self-knowledge can be more challenging than simply data collection. For example, individuals may desire knowledge regarding health assessments and/or recommendations that can be derived from the collected sensor data. As such, artificial intelligence systems, which increasingly include large foundational machine-learned models that have capability to provide health assessments and/or recommendations to the individual based on the collected sensor data, may be implemented to provide the desired knowledge to the individuals. However, wearable technologies may lack computational resources available to store and/or implement each of the distinct artificial intelligence models for performing the specific health assessments and/or recommendations.

Motivated by the need to implement various artificial intelligence models to provide corresponding health assessments and/or recommendations to individuals, the present disclosure is directed to a wearable computing device configured to tokenize sensor data for a wearable computing device so as to facilitate offloading the sensor data from the wearable computing device. Thus, in an embodiment, the wearable computing device is configured to provide the tokenized sensor data to a remote computing device for processing by at least one machine-learned model. In embodiments, the wearable computing device can generate tokenized sensor data such as sensor data tokens or token embeddings in at least one embedding space of the at least one machine-learned model. The tokenized sensor data can be processed by the machine-learned model(s) to provide health assessments and/or recommendations to a user. Offloading the tokenized sensor data from the wearable computing device to the remote computing device for processing can improve accuracy of an output from the machine-learned model(s) by implementing the machine-learned model(s) in a computing device with more computational resources than those of the wearable computing device.

Further, tokenizing the sensor data can provide a relatively high fidelity for the sensor data, which can further improve the accuracy of the output from the machine-learned model(s). In addition, tokenizing the sensor data can assist in ensuring that the collected data relating to the user is safeguarded.

Further, a user may be provided with privacy-related controls allowing the user to make an election as to if and when systems, programs, or features described herein may enable collection of health-related data and/or user information (e.g., information about a user's social network, social actions, or activities, profession, a user's preferences, or a user's current location), and if the user is sent content or communications that may be of a sensitive or private nature from a server. In addition, certain data may be treated in one or more ways before it is stored or used, so that personally identifiable information is removed. For example, a user's identity may be treated so that no personally identifiable information can be determined for the user, or a user's geographic location may be generalized where location information is obtained (such as to a city, ZIP code, or state level), so that a particular location of a user cannot be determined. Thus, the user may have control over what information is collected about the user, how that information is used, and what information is provided to the user. To that end, any information collected as described herein relating to the user (e.g., personal medical data, health conditions, etc.) is capable of being kept private and confidential and not being improperly used or published.

Moreover, one or more security measures can be implemented to ensure that the demographic data and/or physiological data of the user is safeguarded. For example, passcode or fingerprint authentication may be used to control access to the demographic data and the physiological or otherwise personal data of the user. Further, such data of the user can be stored in a privacy enhancing manner and not shared without the express consent of the user. For example, such data can be encrypted to secure the data from unauthorized access.

Example aspects of the present disclosure provide several technical effects, benefits, and/or improvements in computing technology.

### Example Devices and Systems

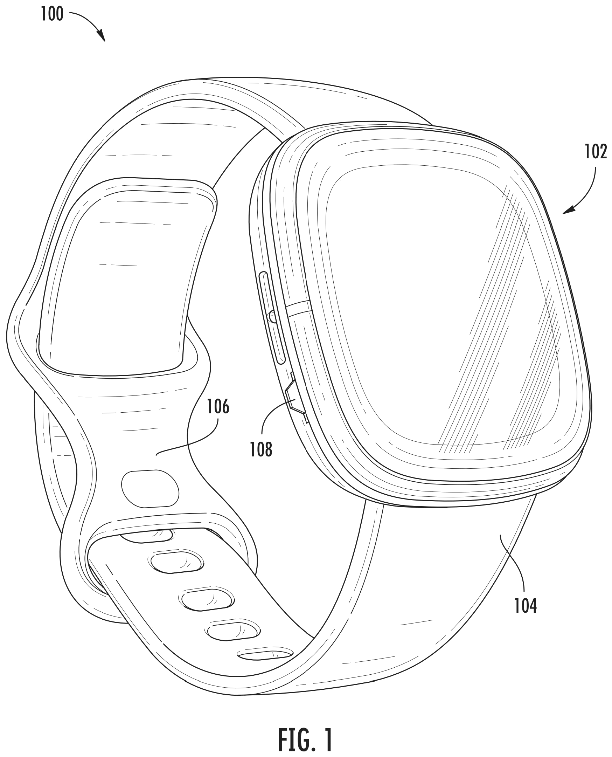

Referring now to the drawings, FIG. 1 illustrates perspective views of an example wearable computing device 100 according to one or more example embodiments of the present disclosure. In example embodiments, the wearable computing device 100 may include, for example, a wearable physiological monitoring device that can be worn by a user 10 and/or capture one or more types of physiological data of the user (e.g., HR data, motion data (e.g., accelerometer data), body temperature data, respiration rate data, blood pressure data, blood oxygenation level data, electrodermal activity (EDA) data, stress related data).

Furthermore, in an embodiment, the wearable computing device 100 may include a display 102, an attachment component 104, a securement component 106, and a button 108 that can be located on a side of the wearable computing device 100. In an embodiment, two sides of the display 102 can be coupled (e.g., mechanically, operatively) to the attachment component 104. In some embodiments, the securement component 106 can be located on, coupled to (e.g., mechanically, operatively), and/or integrated with the attachment component 104. In these or other embodiments, the securement component 106 can be positioned opposite the display 102 on an opposing end of the attachment component 104. In some embodiments, the button 108 can be located on a side of the wearable computing device 100, underneath the display 102.

Furthermore, the display 102 may include any type of electronic display or screen known in the art. For example, in some embodiments, the display 102 may include a liquid crystal display (LCD) or organic light emitting diode (OLED) display such as, for instance, a transmissive LCD display or a transmissive OLED display. Further, the display 102 can be configured to provide brightness, contrast, and/or color saturation features according to display settings that can be maintained by control circuitry and/or other internal components and/or circuitry of the wearable computing device 100. In some embodiments, the display 102 may include a touchscreen such as, for instance, a capacitive touchscreen. For example, in these embodiments, the display 102 may include a surface capacitive touchscreen or a projective capacitive touch screen that can be configured to respond to contact with electrical charge-holding members or tools, such as a human finger. While the wearable computing device 100 is shown in example embodiments of the present disclosure to have the display 102, it should be understood that, in some embodiments, the wearable computing device 100 does not have any type of display unit.

In some embodiments, the display 102 can be configured to provide (e.g., render) a variety of information such as, for example, the time, the date, physiological data of a user wearing the wearable computing device 100, readings based upon user input, and/or other information. In an embodiment, the physiological data can include, but are not limited to, HR data (e.g., heart beats per minute), motion data (e.g., movement data, accelerometer data), blood pressure data, body temperature data, respiration rate data, blood oxygenation level data, EDA data, stress related data and/or any other physiological data that one of ordinary skill in the art would understand that can be measured by the wearable computing device 100. In some embodiments, the readings based upon user input can include, but are not limited to, activities performed by the user, a sleep schedule of the user, and/or any other metric that one of ordinary skill in the art would understand that can be input by a user into the wearable computing device 100.

The attachment component 104 can be used to attach (e.g., affix, fasten) the wearable computing device 100 to a user thereof (e.g., to the user's 10 body or clothing). In some embodiments, the attachment component 104 can take the form of, for example, a strap, an elastic band, a rope, and/or any other form of attachment one of ordinary skill in the art would understand can be used to attach the wearable computing device 100 to a user. For example, the wearable computing device 100 can be configured as a wrist bracelet, watch, ring, electrode, finger-clip, toe-clip, chest-strap, ankle strap, and/or a device placed in a pocket. In additional or alternative embodiments, the wearable computing device 100 can be embedded in something in contact with the user 10 such as, for instance, clothing, a mat that can be positioned under the user 10, a blanket, a pillow, and/or another accessory.

The securement component 106 can facilitate attachment of attachment component 104 upon a user of wearable computing device 100. In some embodiments, the securement component 106 can include, but is not limited to, a pin and hole locking mechanism (e.g., a buckle), a magnet system, a lock, a clip, and/or any other type of securement that one of ordinary skill would understand can be used to facilitate attachment of the wearable computing device 100 to a user. In an embodiment, the wearable computing device 100 does not include the securement component 106. For example, in an embodiment, the wearable computing device 100 can be secured to a user with a strap that can be tied around the user's wrist and/or another suitable appendage.

The button 108 can allow for a user to interact with the wearable computing device 100 and/or allow for the user to provide a form of input into wearable computing device 100. For instance, as described above, in example embodiments, the wearable computing device 100 can include a screen such as, for example, a touch screen that can receive inputs through (e.g., by way of) the touch of the user. In additional or alternative embodiments, the wearable computing device 100 can include a microphone that can receive inputs through (e.g., by way of) voice commands of a user.

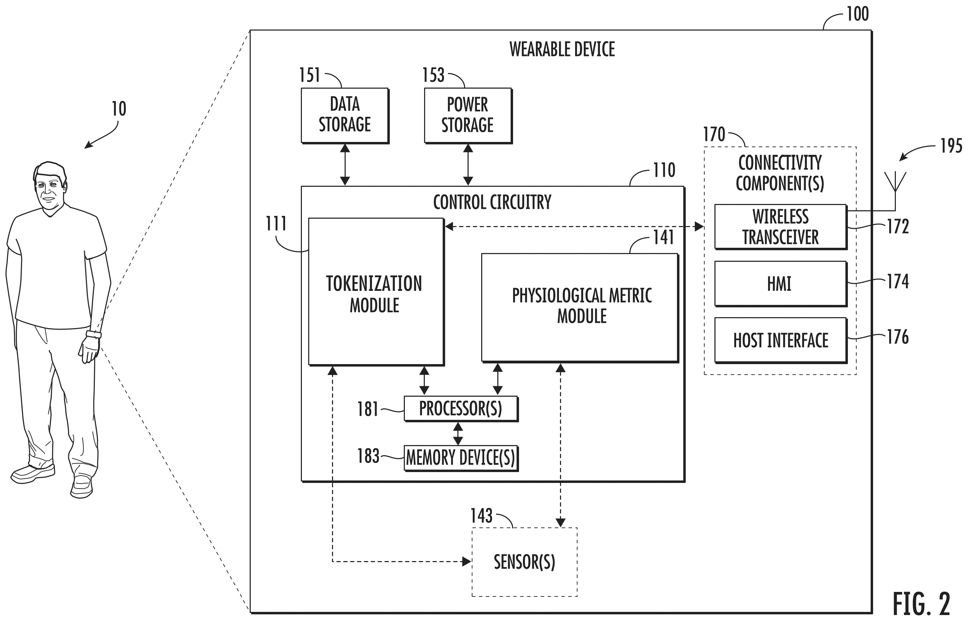

Referring now to FIG. 2, a block diagram of the wearable computing device 100 according to one or more example embodiments of the present disclosure is illustrated. That is, for instance, FIG. 2 illustrates a block diagram of one or more internal and/or external components of the wearable computing device 100 according to one or more example embodiments of the present disclosure.

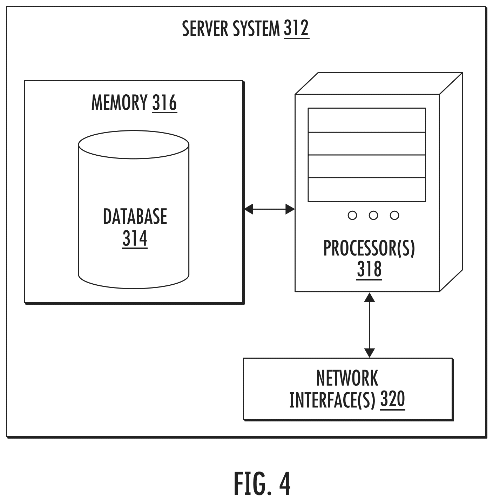

Although certain embodiments are disclosed herein in the context of wearable physiological monitoring devices, it should be appreciated that the present disclosure is not so limiting. For example, it should be understood that the physiological monitoring principles and features disclosed herein can be performed and/or implemented using any suitable type of computing device or combination of computing devices such as, for example, a client computing device, a laptop, a tablet, a server (e.g., a server system 312 described below and depicted in FIG. 4), the wearable computing device 100, a mobile computing device 304, such as a smartphone, and/or another computing device, whether wearable or not.

As shown in FIG. 2, the wearable computing device 100 can be worn by the user 10 and/or can be configured to gather data regarding activities performed by user 10 and/or a physiological state of the user 10. In some embodiments, the data may include motion data regarding the user's movements and/or physiological data obtained by measuring various physiological characteristics of the user 10 (e.g., heart rate, respiratory data, body temperature, blood oxygen levels, perspiration levels, movement data).

Further, as shown, the wearable computing device 100 can include control circuitry 110. Although certain modules and/or components are illustrated as part of the control circuitry 110 in the diagram of FIG. 2, it should be understood that the control circuitry 110 associated with the wearable computing device 100 and/or other components or devices in accordance with example embodiments of the present disclosure can include additional components and/or circuitry such as, for instance, one or more additional components of the illustrated components depicted in FIG. 2. Furthermore, in certain embodiments, one or more of the illustrated components of the control circuitry 110 can be omitted and/or different than that shown in FIG. 2 and described in association therewith.

The term "control circuitry" is used herein according to its broad and/ordinary meaning and can include any combination of software and/or hardware elements, devices, and/or features that can be implemented in connection with operation of the wearable computing device 100. Furthermore, the term "control circuitry" can be used substantially interchangeably in certain contexts herein with one or more of the terms "controller," "integrated circuit," "IC," "application-specific integrated circuit," "ASIC," "controller chip," or the like.

The control circuitry 110 may include one or more processors 181, one or more memory devices 183, and/or electrical connections. In an embodiment, the control circuitry 110 can be implemented on a system on a chip (SoC), however, those skilled in the art will recognize that other hardware and/or firmware implementations are possible.

In one or more embodiments, the processor(s) 181 can be configured to execute computer-readable instructions that, when executed, cause the wearable computing device 100 to perform one or more operations. In at least an embodiment, the processor(s) 181 can be configured to execute operational code (e.g., instructions, processing threads, software) for the wearable computing device 100 such as, for instance, firmware or the like. In the example embodiment depicted in FIG. 2, the processor(s) 181 can each be a central processing unit (CPU), microprocessor, microcontroller, integrated circuit (e.g., an application-specific integrated circuit (ASIC)), and/or another type of processing device. In this or another example embodiment, the processor(s) 181 can be coupled to (e.g., electrically, communicatively, physically, operatively) to one or more components of the control circuitry 110 and/or the wearable computing device 100 such that the processor(s) 181 can facilitate one or more operations in accordance with the embodiments described herein.

In an embodiment, as shown in FIG. 2, the computer-readable instructions and/or operational code that can be executed by the processor(s) 181 can be stored in one or more data storage devices of the wearable computing device 100, such as the memory device(s) 183 of the wearable computing device 100. In some embodiments, the memory device(s) 183 can be coupled to (e.g., electrically, communicatively, physically, operatively) to one or more components of the control circuitry 110 and/or the wearable computing device 100 such that the memory device(s) 183 can facilitate one or more operations in accordance with the embodiments described herein.

The memory device(s) 183 can store computer-readable and/or computer executable entities (e.g., data, information, applications, models, algorithms) that can be created, modified, accessed, read, retrieved, and/or executed by each of the processor(s) 181. In some embodiments, the memory device(s) 183 can constitute, include, be coupled to (e.g., operatively), and/or otherwise be associated with a computing system and/or media such as, for example, one or more computer-readable media, volatile memory, non-volatile memory, random-access memory (RAM), read only memory (ROM), hard drives, flash drives, and/or other memory devices. In these or other embodiments, such one or more computer-readable media can include, constitute, be coupled to (e.g., operatively), and/or otherwise be associated with one or more non-transitory computer-readable media.

Still referring to FIG. 2, the control circuitry 110 may include a tokenization module 111, a physiological metric module 141, and/or other modules and/or data that can be used to facilitate one or more operations described herein. The tokenization module 111 may include one or more hardware and/or software components and/or features that can be configured to tokenize sensor data, as described further below.

The wearable computing device 100 can further include one or more sensors 143 communicatively coupled to the tokenization module 111 and/or the physiological metric module 141. In embodiments, the sensor(s) 143 may include an inertial measurement unit (IMU) which includes one or more accelerometers and/or one or more gyroscopes. The accelerometer(s) may be used to capture motion information with respect to the wearable computing device 100. The gyroscope(s) may also be used additionally or alternatively to capture motion information with respect to the wearable computing device 100. For example, the IMU may be configured as a six-axis or six-dimensional inertial measurement unit (e.g., a tri-axial accelerometer and a tri-axial gyroscope). The motion information obtained via the IMU may be associated with the user when the wearable computing device 100 is worn or carried by the user. The IMU may further include one or more magnetometers, one or more barometers, and/or any other suitable sensors.

In additional or alternative embodiments, the sensor(s) 143 may include physiological sensors that can be configured to collect the physiological data of the user in accordance with various embodiments disclosed herein. For example, the physiological sensors may include a heart rate sensor, photoplethysmography (PPG) sensor, and/or other physiological sensors. In some embodiments, the physiological sensor(s) can be disposed on, coupled to, embedded and/or integrated in, and/or otherwise be associated with the wearable computing device 100 such that the physiological sensor(s) can be in contact with or substantially in contact with human skin when the wearable computing device 100 is worn by a user. For example, in embodiments the physiological sensor(s) can be disposed on, coupled to, and/or otherwise be associated with an interior or skin-side of the wearable computing device 100 (e.g., a side of the wearable computing device 100 that contacts, touches, and/or faces the skin of the user). In additional and/or alternative embodiments, the wearable computing device 100 can be configured to receive the physiological data of the user from one or more physiological sensors external to (e.g., not embedded and/or integrated in) the wearable computing device 100.

In an embodiment, as mentioned, the physiological metric module 141 can, for example, be communicatively coupled with the sensor(s) 143 such that the physiological metric module 141 can receive the data of the user 10 collected by the sensor(s) 143. The physiological metric module 141 can, for example, calculate physiological metrics, including but not limited to, the cardiac metric(s), respiratory metric(s), neurological metric(s), musculoskeletal metric(s), etc., of the user 10 based on the data of the user 10 (e.g., according to known physiological metric calculations). For example, the physiological metric module 141 may include a machine-learned model trained to receive the sensor data as input and to generate the physiological metrics and/or assessments as output. In additional or alternative embodiments, the wearable computing device 100 can be configured to communicate with another computing device or server that can analyze and/or interpret the collected sensor data (e.g., according to known physiological metric calculations), as described further below.

In the example embodiment depicted in FIG. 2, the wearable computing device 100 can include one or more data storage components 151 (denoted in FIG. 2 as "data storage 151") communicatively coupled to the control circuitry 110. The data storage component(s) 151 may include any suitable or desirable type of data storage such as, for instance, solid-state memory, which can be volatile or non-volatile. In some embodiments, such solid-state memory of the wearable computing device 100 may include any of a wide variety of technologies such as, for instance, flash integrated circuits, phase change (PC) memory, phase change (PC) random-access memory (RAM), programmable metallization cell RAM (PMC-RAM or PMCm), ovonic unified memory (OUM), resistance RAM (RRAM), NAND memory, NOR memory, EEPROM, ferroelectric memory (FeRAM), MRAM, or other discrete NVM (non-volatile solid-state memory) chips. In some embodiments, the data storage component(s) 151 can be used to store system data, such as operating system data and/or system configurations or parameters. In some embodiments, the wearable computing device 100 can include data storage utilized as a buffer and/or cache memory for operational use by the control circuitry 110.

The data storage component(s) 151 can include various sub-modules that can be implemented to facilitate the physiological monitoring and the cardiac assessment principles and features disclosed herein. For example, in at least an embodiment, the data storage component(s) 151 can include one or more sub-modules that can include, but not limited to: an information collection module (e.g., the physiological metric module 141) that can manage the collection of the physiological data, demographic data, and/or anthropometric data; a heart rate determination module that can determine values and/or patterns of one or more types of heart rates of the user 10; the tokenization module 111; a sleep detection module that can detect an attempt or onset of sleep by the user 10; a presentation module that can manage presentation of information to the user 10 that can be associated with the cardiac assessment; a feedback management module for collecting and interpreting any input data and/or feedback received from the user 10; and/or another sub-module.

The wearable computing device 100 can further include a power storage module 153 (denoted in FIG. 2 as "power storage 153"), which may include a rechargeable battery, one or more capacitors, or other charge-holding device(s). In some embodiments, the power stored by the power storage module 153 can be utilized by the control circuitry 110 for operation of the wearable computing device 100, such as for powering the display 102. In some embodiments, the power storage module 153 can receive power over a host interface of the wearable computing device 100 (e.g., via one or more host interface circuitry and/or components 176 (denoted as "host interface 176" in FIG. 2)) and/or through other means.

The wearable computing device 100 can further include one or more connectivity components 170, which can include, for example, a wireless transceiver 172. The wireless transceiver 172 can be communicatively coupled to one or more antenna devices 195, which can be configured to wirelessly transmit and/or receive data and/or power signals to and/or from the wearable computing device 100 using, but not limited to, peer-to-peer, WLAN, and/or cellular communications. For example, the wireless transceiver 172 can be utilized to communicate data and/or power between the wearable computing device 100 and an external computing device, which can be configured to interface with the wearable computing device 100. In certain embodiments, the host interface 176 can include, for example, wired and/or wireless interface components that can communicatively couple the wearable computing device 100 with the external computing device to receive data and/or power therefrom and/or transmit data thereto. The host interface 176 according to example embodiments can utilize and/or otherwise be associated with any suitable or desirable communication protocol and/or physical connector such as, for instance, universal serial bus (USB), micro-USB, Wi-Fi, Bluetooth, FireWire, PCIe, or the like. For wireless connections, the host interface 176 according to example embodiments can be incorporated with the wireless transceiver 172.

Moreover, as shown in FIG. 2, the connectivity component(s) 170 can further include one or more HMIs 174 that can be used by the wearable computing device 100 to receive input data from user 10 and/or provide output data to the user 10. For instance, in some embodiments, the HMI 174 of the wearable computing device 100 may include a touchscreen display that can be configured to provide (e.g., render) output data to the user 10 and/or to receive user input through user contact with the touchscreen display. In some embodiments, the HMI(s) 174 can further constitute and/or include one or more buttons or other input/output components or features.

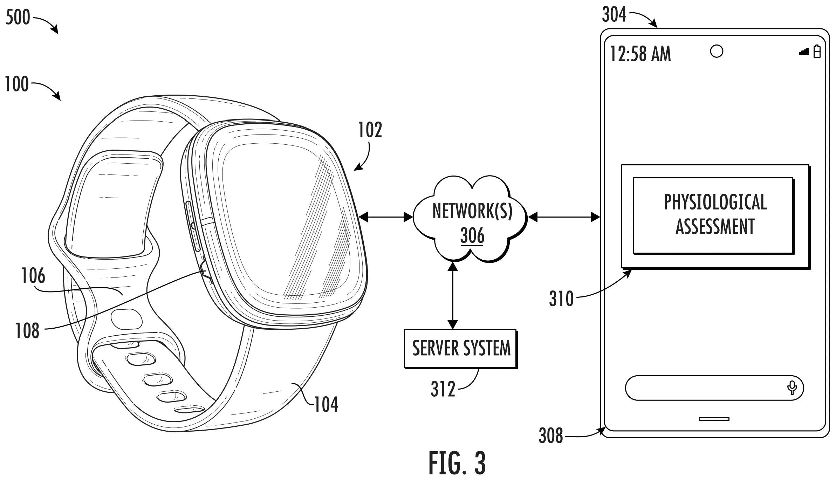

Referring now to FIG. 3, a diagram of an example computing environment 300 according to one or more example embodiments of the present disclosure is illustrated. As shown, the computing environment 300 depicted in FIG. 3 illustrates an example networked relationship between the wearable computing device 100 and a remote computing device, such as a mobile computing device 304 and/or a server system 312, in accordance with one or more embodiments.

With reference to the example embodiment described above and depicted in FIG. 2, the wearable computing device 100 according to example embodiments of the present disclosure can perform analyze and/or interpret a portion of the sensor data for the user 10 and/or perform operation(s) to based on the portion of the sensor data. As such, in certain embodiments the wearable computing device 100 can be capable of and/or configured to collect the portion of the sensor data of the user 10 and/or perform operation(s) using such readings.

However, the wearable computing device 100 may include one or more infrastructure constraints that limit the capabilities of the wearable computing device 100 to analyze and/or interpret all of the sensor data for the user 10 and/or perform operation(s) based on all of the sensor data. As used herein, an "infrastructure constraint" generally refers to a variable representing available resources for the wearable computing device 100 to perform desired operations. Thus, in an embodiment, the infrastructure constraint(s) may include energy storage capabilities, available data storage to store sensor data, available computational resources for executing machine-learned models to analyze and/or interpret the sensor data, and/or any other suitable resource that may limit execution of the wearable computing device 100.

As such, the wearable computing device 100 can be in communication with the remote computing device 304, 312 that has more computational resources available than the wearable computing device 100 to analyze and/or interpret all of the sensor data. Due to the infrastructure constraint(s) of the wearable computing device 100, the wearable computing device 100 may be unable to communicate raw sensor data to the remote computing device 304, 312. As such, the wearable computing device 100 may be configured to generate tokenized sensor data via the tokenization module 111, as will be described further below, that can be communicated to remote computing devices 304, 312 given the infrastructure constraint(s) of the wearable computing device 100.

Thus, the wearable computing device 100 can relay the tokenized sensor data to the remote computing device 304, 312 over one or more networks 306 to other devices. This includes, in some embodiments, relaying the tokenized sensor data to devices capable of serving as Internet-accessible data sources, thus permitting the collected data to be viewed, for example, using a web browser or network-based application at, for instance, the remote computing device 304, 312. For example, the wearable computing device 100, e.g., while being worn by the user 10, can capture, calculate, and/or store the sensor data of the user 10 using the sensor(s) 143. The wearable computing device 100 can then transmit (e.g., periodically or continuously) tokenized sensor data representative of the sensor data over the network(s) 306 to the mobile computing device 304 and/or a server system 312 where the data can be stored, processed, and visualized by the user 10 and/or another entity (e.g., a health care professional). Accordingly, in an embodiment, the mobile computing device 304 may be configured to generate an intelligent notification 310 and provide the intelligent notification 310 to the user 10, e.g., via a display 308 or a second computing device. In some embodiments, the intelligent notification 310 can include a physiological assessment, which may be output by a machine-learned model programmed in the remote computing device 304, 312.

In one or more embodiments, the communication between the wearable computing device 100 and the mobile computing device 304 can be facilitated by the network(s) 306. In some embodiments, the network(s) 306 may include, for instance, one or more of an ad hoc network, a peer-to-peer communication link, an intranet, an extranet, a virtual private network (VPN), a local area network (LAN), a wireless LAN (WLAN), a wide area network (WAN), a wireless WAN (WWAN), a metropolitan area network (MAN), a portion of the Internet, a portion of the public switched telephone network (PSTN), a cellular telephone network, and/or any other type of network. In some embodiments, the communication between the wearable computing device 100 and the mobile computing device 304 can also be performed through a direct wired connection. In these or other embodiments, this direct-wired connection can be associated with any suitable or desirable communication protocol and/or physical connector such as, for instance, universal serial bus (USB), micro-USB, Wi-Fi, Bluetooth, FireWire, PCIe, or the like.

In example embodiments, a variety of computing devices can be in communication with the wearable computing device 100 to facilitate the user's cardiac assessment and/or alteration (e.g., improvement). Although the mobile computing device 304 is depicted as a smartphone in the example embodiment illustrated in FIG. 3, it should be understood that the present disclosure is not so limiting. For instance, the mobile computing device 304 according to example embodiments may include, for example, a smartphone with the display 308 as depicted in FIG. 3, a personal digital assistant (PDA), a mobile phone, a tablet, a personal computer, a laptop computer, a smart television, a video game console, and/or another computing device that can be external to the wearable computing device 100.

The remote computing device 304, 312 can be configured to use the tokenized sensor data of the user 10 to determine physiological metric(s) of the user 10 and/or perform physiological assessment(s) of the user 10 based on the sensor data. For example, the remote computing device 304, 312 may include one or more machine-learned models that can include at least one physiological assessment model. In such embodiments, the physiological assessment model(s) is configured to generate one or more physiological metrics and/or assessments for the user using machine learning. For example, in an embodiment, the machine-learned model(s) may be trained to output the physiological metric(s) and/or assessment(s) in response to receiving the tokenized sensor data of the user 10. More particularly, the remote computing device 304, 312 includes more computational resources than the wearable computing device 100, which permits the remote computing device 304, 312 to determine the physiological metric(s) and/or assessment(s) in a more compute efficient manner.

The machine-learned model can be or include one or multiple machine-learned models or model components. Example machine-learned models can include neural networks (e.g., deep neural networks) or a generative model (e.g., large language models (LLM), nonlinear models or linear models, decision tree based models, support vector machines, hidden Markov models, Bayesian networks, and/or k-means clustering models, etc. Example machine-learned models can also use other architectures in lieu of or in addition to those models specifically mentioned herein.

Neural networks, such as those described herein, can include feed-forward neural networks, recurrent neural networks (e.g., long short-term memory recurrent neural networks), convolutional neural networks, and/or other forms of neural networks. Some example machine-learned models can leverage an attention mechanism such as self-attention. For example, some example machine-learned models can include multi-headed self-attention models (e.g., transformer models). In another embodiment, the machine learning models described herein may include a rule-based approach, wherein actions are chosen based on a predetermined set of if-then rules or mathematical expressions with pre-defined parameters.

Referring particularly to FIGS. 3 and 4, the wearable computing device 100 can transmit the tokenized sensor data of the user 10 to the server system 312 (e.g., via the network(s) 306). In this embodiment, the server system 312 can analyze the received tokenized sensor data to perform the physiological assessment and/or can use the received tokenized sensor data to update a user profile for the user 10 that can be stored in a database 314 (e.g., a log) of a memory 316 (FIG. 4) of the server system 312.

In some embodiments, the server system 312 can be implemented on one or more standalone data processing apparatuses or a distributed network of computers. In some embodiments, the server system 312 can employ various virtual devices and/or services of third-party service providers (e.g., third-party cloud service providers) to provide the underlying computing resources and/or infrastructure resources of the server system 312. In some embodiments, the server system 312 can include, but is not limited to, a handheld computer, a tablet computer, a laptop computer, a desktop computer, or a combination of any two or more of these data processing devices or other data processing devices.

Referring particularly to FIG. 4, the server system 312 can include one or more processors 318 such as, for instance, one or more CPUs. In these or other embodiments, the server system 312 can include one or more network interfaces 320 that can include, for example, an input/output (I/O) interface to the mobile computing device 304 and/or the wearable computing device 100. In some embodiments, the server system 312 can include one or more communication buses for interconnecting these components.

The memory 316 according to example embodiments can include high-speed random-access memory such as, for instance, DRAM, SRAM, DDR RAM, or other random-access solid-state memory devices; and, optionally, can include non-volatile memory such as, for example, one or more magnetic disk storage devices, one or more optical disk storage devices, one or more flash memory devices, or one or more other non-volatile solid state storage devices. The memory 316, optionally, can include one or more storage devices that can be remotely located from the processor(s) 318 (e.g., processing unit(s)). Further, the memory 316, or alternatively the non-volatile memory within the memory 316, can include a non-transitory computer readable storage medium. In some embodiments, the memory 316, or the non-transitory computer readable storage medium of the memory 316, can store one or more programs, modules, and data structures. In these embodiments, such programs, modules, and data structures can include, but not be limited to, one or more of an operating system that can include procedures for handling various basic system services and for performing hardware dependent tasks.

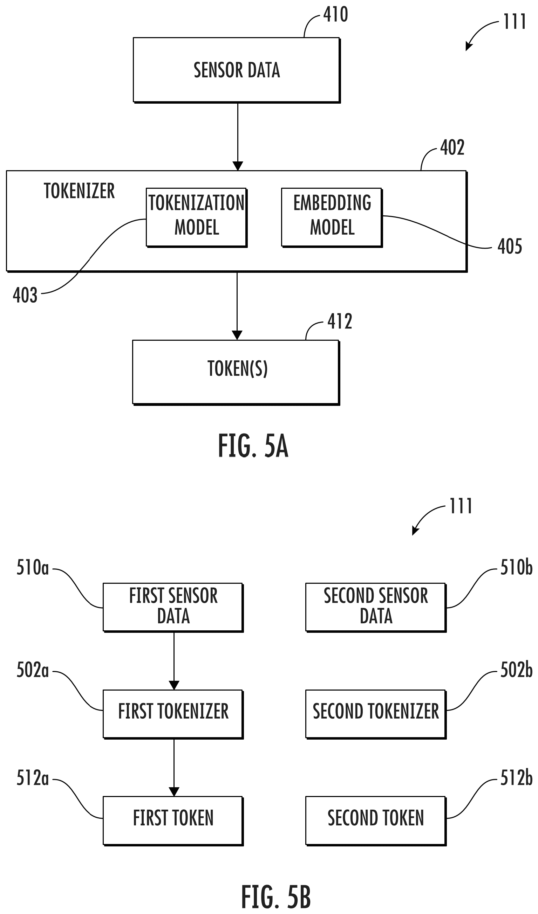

Referring now to FIGS. 5A and 5B, a block diagram of an embodiment of the tokenization module 111 according to the present disclosure is illustrated. The wearable computing device 100 can be configured to collect the sensor data 410 of the user 10 using embedded sensors and/or external devices, as mentioned above. In some embodiments, the sensor data 410 can optionally be pre-processed by a processing system (not shown). Various types of pre-processors and pre-processing can be provided for different implementations. By way of example, the sensor data 410 can be pre-processed to generate textual representations that can be tokenized into text tokens for processing by a machine-learned model programmed in the remote computing device 304, 312. For example, the sensor data 410 can be provided to a machine-learned classification model that is configured to generate one or more predictions or classifications of the raw sensor data 410. A prediction or classification can be represented as text which can be provided to a text tokenizer for generating one or more text tokens as input to the machine-learned model. For instance, the sensor data 410 can be provided as input to a machine-learned classification or prediction model that is configured to determine contextual features. The model can output a text representation of the contextual features. The contextual features can include information about the context around and/or near to a user and/or information about the context of a user directly, such as a user's location, activity level, etc.

The sensor data 410 can then be provided to a tokenizer 402. The tokenizer 402 may include one or more hardware and/or software components and/or features that can be configured to perform the operations described herein. The tokenizer 402 can generate tokenized sensor data from the sensor data 410 (e.g., via vector quantization algorithms). The tokenized sensor data includes the sensor data 410 compressed into a compressed bitstream. For instance, the tokenization module 111 can generate tokens 412 that embed and compress sensor data 410 into representations capable of transmission via the network 306 given the infrastructure constrain(s) of the wearable computing device 100. Further, the tokens 412 may be capable of processing by the machine-learned model programmed in the remote computing device 304, 312. In additional or alternative embodiments, the tokenizer 402 can generate segments, chunks, or other subsets of data from the sensor data capable of transmission via the network 306 given the infrastructure constrain(s) of the wearable computing device 100.

In example embodiments, the tokenizer 402 can include one or more machine-learned tokenization models 403 that are configured to generate one or more sensor data tokens 412 as an output in response to the sensor data 410 as an input. In some examples, the machine-learned tokenization model(s) 403 can embed the sensor data token(s) 412 into an embedding space for the machine-learned model programmed in the remote computing device 304, 312. An embedding model can compress and represent the token(s) 412 as token embedding(s). The machine-learned tokenization model(s) 403 can be configured to generate sensor data token(s) 412 that are adapted for processing in a common embedding space of the machine-learned model programmed in the remote computing device 304, 312. In embodiments, the machine-learned tokenization model(s) 403 can generate token embedding(s) by embedding the sensor data token(s) 412 into an embedding space of the machine-learned model programmed in the remote computing device 304, 312. For example, the tokenizer 402 can include one or more machine-learned embedding models 405 such as a transformer or encoder configured to embed the sensor data token(s) 412 into the embedding space of the machine-learned model programmed in the remote computing device 304, 312. In example embodiments, the embedding space of the machine-learned model programmed in the remote computing device 304, 312 can be a latent embedding space, a text embedding space, an image embedding space, and/or an audio embedding space.

The machine-learned tokenization model(s) 403 or the embedding model(s) 405 can generate token embedding(s) from the sensor data 410. The tokens 412 or token embedding(s) can contain compressed sensor data 410 that can be transmitted in a compute efficient manner. The machine-learned tokenization model(s) 403 and/or the embedding model(s) 405 can extract contextual features from the sensor data 410 which can be compressed for representation as token(s) and/or embedding(s). The embedding model(s) 405 can include one or more embedding layers configured to receive features or sensor data 410 and generate tokenized sensor data such as the sensor data token(s) 412. The embedding layers can include radar embedding layer(s) (e.g., a transformer) and text embedding layer(s) (e.g., a word embedding layer). The model can include a transformer encoder to generate radar, lidar, optical, ultrasonic, and/or other sensor modality representations.

The tokenization module 111 can include one or more tokenizers 402, 502a, 502b for tokenizing sensor data 410, 510a, 510b for different sensing modalities. In an example embodiment, the tokenization module 111 can include a single tokenizer 402 that is configured to generate sensor data token(s) 412 from different types of sensors, as shown in FIG. 5A. For example, the single tokenizer 402 can be configured to generate one or more tokens 412 from sensor data 410 of sensors of multiple types (e.g., ultrasonic sensor, radar sensor, etc.). In another example embodiment, the tokenization module 111 can include different tokenizers 502a, 502b for different types of sensors, as shown in FIG. 5B. For instance, a first tokenizer 502a can be configured to generate one or more first tokens 512a from first sensor data 510a of a sensor of a first type (e.g., ultrasonic sensor), and a second tokenizer 512b can be configured to generate one or more second tokens 512b from second sensor data 510b of a sensor of a second, different type (e.g., radar sensor). As such, the tokenizer(s) 402, 502a, 502b of the tokenization module 111 can include a machine-learned tokenization model that is configured to generate sensor data tokens 412, 512a, 512b in response to sensor data 410, 510a, 510b of one or more sensor modalities.

### Example Methods

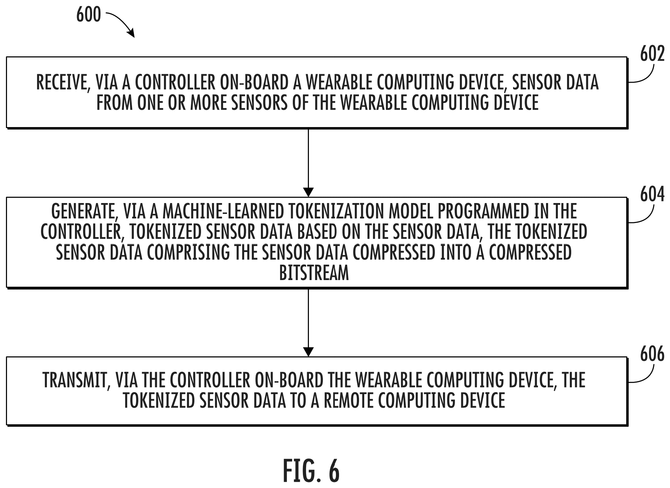

FIG. 6 illustrates a flow diagram of an embodiment of a computer-implemented method 600 according to the present disclosure. The computer-implemented method 600 can be implemented using, for instance, the wearable computing device 100, the mobile computing device 304, and/or the server system 312 described above with reference to the example embodiments depicted in FIGS. 1-5.

The example embodiment illustrated in FIG. 6 depicts operations performed in a particular order for purposes of illustration and discussion. Those of ordinary skill in the art, using the disclosures provided herein, will understand that various operations or steps of the computer-implemented method 600 or any of the other methods disclosed herein can be adapted, modified, rearranged, performed simultaneously, include operations not illustrated, and/or modified in various ways without deviating from the scope of the present disclosure.

As shown at (602), the computer-implemented method 600 may include receiving, by the wearable computing device 100, data from one or more sensors. The sensor data may include, but is not limited to, motion data and/or physiological data for a user, as discussed above.

As shown at (604), the computer-implemented method 600 may include generating, via a machine-learned tokenization model programmed in the wearable computing device 100, tokenized sensor data based on the sensor data. The tokenized sensor data may include compressed sensor data capable of being transmitted via one or more networks while satisfying infrastructure constraint(s) of the computing device, as discussed above.

As shown at (606), the computer-implemented method 600 may include transmitting, by the wearable computing device 100, the tokenized sensor data to a remote computing device. The remote computing device may include the mobile computing device 304, and/or the server system 312, as discussed above. As such, the remote computing device can analyze and/or interpret the sensor data in a compute efficient manner (e.g., due to having relatively more available computational resources).

### Additional Disclosure

The technology discussed herein makes reference to servers, databases, software applications, and other computer-based systems, as well as actions taken and information sent to and from such systems. The inherent flexibility of computer-based systems allows for a great variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

While the present subject matter has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations or additions to the present subject matter as would be readily apparent to one of ordinary skill in the art. For instance, features illustrated or described as part of one embodiment can be used with another embodiment to yield a still further embodiment. Thus, it is intended that the present disclosure cover such alterations, variations, and equivalents.

Aspects of the disclosure have been described in terms of illustrative embodiments thereof. Any and all features in the following claims can be combined or rearranged in any way possible, including combinations of claims not explicitly enumerated in combination together, as the example claim dependencies listed herein should not be read as limiting the scope of possible combinations of features disclosed herein. Accordingly, the scope of the present disclosure is by way of example rather than by way of limitation, and the subject disclosure does not preclude inclusion of such modifications, variations or additions to the present subject matter as would be readily apparent to one of ordinary skill in the art. Moreover, terms are described herein using lists of example elements joined by conjunctions such as "and," "or," "but," etc. It should be understood that such conjunctions are provided for explanatory purposes only. Clauses and other sequences of items joined by a particular conjunction such as "or," for example, can refer to "and/or," "at least one of", "any combination of" example elements listed therein, etc. Terms such as "based on" should be understood as "based at least in part on."

The term "can" should be understood as referring to a possibility of a feature in various implementations and not as prescribing an ability that is necessarily present in every implementation. For example, the phrase "X can perform Y" should be understood as indicating that, in various implementations, X has the potential to be configured to perform Y, and not as indicating that in every instance X must always be able to perform Y. It should be understood that, in various implementations, X might be unable to perform Y and remain within the scope of the present disclosure.

The term "may" should be understood as referring to a possibility of a feature in various implementations and not as prescribing an ability that is necessarily present in every implementation. For example, the phrase "X may perform Y" should be understood as indicating that, in various implementations, X has the potential to be configured to perform Y, and not as indicating that in every instance X must always be able to perform Y. It should be understood that, in various implementations, X might be unable to perform Y and remain within the scope of the present disclosure.

## Claims

1. A method for controlling a wearable computing device, the method comprising:
receiving, via a controller, sensor data from one or more sensors of the wearable computing device;
generating, via a machine-learned tokenization model programmed in the controller on-board the wearable computing device, tokenized sensor data based on the sensor data, the tokenized sensor data comprising the sensor data compressed into a compressed bitstream; and
transmitting, via the controller on-board the wearable computing device, the tokenized sensor data to a remote computing device.

2. The method of claim 1, wherein the tokenized sensor data is configured to be transmitted via a communication protocol while satisfying an infrastructure constraint of the wearable computing device.

3. The method of claim 1 or 2, wherein the tokenized sensor data is configured for at least one embedding space of a machine-learned model programmed in a remote controller of the remote computing device.

4. The method of claim 3, wherein the tokenized sensor data comprises one or more token embeddings, and wherein the method further comprises:
generating one or more sensor data tokens; and
generating the one or more token embeddings by embedding the one or more sensor data tokens in an embedding space of the machine-learned model.

5. The method of any one of the preceding claims, wherein:
the sensor data comprises first sensor data from a first sensor of a first sensor type and second sensor data from a second sensor of a second sensor type;
the machine-learned tokenization model comprises a first tokenizer configured to tokenize the first sensor data and a second tokenizer configured to tokenize the second sensor data; and
generating, via the machine-learned tokenization model programmed in the controller, tokenized sensor data based on the sensor data further comprises:
generating, via the first tokenizer, a first token based on the first sensor data; and
generating, via the second tokenizer, a second token based on the second sensor data.

6. The method of any one of claims 1 to 4, wherein:
the sensor data comprises first sensor data from a first sensor of a first sensor type and second sensor data from a second sensor of a second sensor type;
the machine-learned tokenization model comprises a tokenizer configured to tokenize the first sensor data and the second sensor data; and
generating, via the machine-learned tokenization model programmed in the controller, tokenized sensor data based on the sensor data further comprises:
generating, via the tokenizer, a first token based on the first sensor data and a second token based on the second sensor data.

7. The method of any one of the preceding claims, further comprising identifying, via a model programmed in the controller, a physiological metric for a user based on the sensor data.

8. The method of any one of the preceding claims, wherein the tokenized sensor data comprises one or more sensor data tokens.

9. The method of any one of the preceding claims, wherein the remote computing device comprises a user computing device or a server computing device.

10. The method of any one of the preceding claims, wherein the wearable computing device comprises a smart watch.

11. A wearable computing device, comprising:
a controller comprising at least one processor, the at least one processor configured to perform a plurality of operations, the plurality of operations comprising:
receiving sensor data from one or more sensors of the wearable computing device;
generating, via a machine-learned tokenization model programmed in the controller, tokenized sensor data based on the sensor data, the tokenized sensor data comprising the sensor data compressed into a compressed bitstream; and
transmitting the tokenized sensor data to a remote computing device.

12. The wearable computing device of claim 11, wherein the tokenized sensor data is configured to be transmitted via a communication protocol while satisfying an infrastructure constraint of the wearable computing device.

13. The wearable computing device of claim 11 or 12, wherein the tokenized sensor data is configured for at least one embedding space of a machine-learned model programmed in a remote controller of the remote computing device.

14. The wearable computing device of claim 13, wherein the tokenized sensor data comprises one or more token embeddings, and wherein the plurality of operations further comprise:
generating one or more sensor data tokens; and
generating the one or more token embeddings by embedding the one or more sensor data tokens in an embedding space of the machine-learned model.

15. The wearable computing device of any one of claims 11 to 14, wherein the wearable computing device comprises a smart watch.
